# EUROPÄISCHE PATENTSCHRIFT

(11) **EP 3 028 623 B1**
(45) Veröffentlichungstag und Bekanntmachung des Hinweises auf die Patenterteilung: **01.08.2018**
(21) Anmeldenummer: 15192242.4
(22) Anmeldetag: 30.10.2015
(51) Int. Cl.: A61B 1/00, A61B 1/018, A61B 17/00, A61B 10/04, A61B 17/29

(54) **ENDOSKOPISCHES INSTRUMENT SOWIE ENDOSKOPISCHES INSTRUMENTENSYSTEM**
ENDOSCOPIC INSTRUMENT AND ENDOSCOPIC INSTRUMENT SYSTEM
INSTRUMENT ENDOSCOPIQUE ET SYSTEME D'INSTRUMENT ENDOSCOPIQUE

(30) Priorität: 05.12.2014 DE 102014118003
(43) Veröffentlichungstag der Anmeldung: 08.06.2016
(73) Patentinhaber: Karl Storz SE & Co. KG, 78532 Tuttlingen (DE)
(72) Erfinder: Preis, Fridolin, 78532 Tuttlingen (DE)

(56) Entgegenhaltungen:
- EP-A2- 0 455 188
- US-A- 5 392 765
- US-A1- 2010 145 142
- US-A1- 2012 035 416
- US-A1- 2014 288 460

## Beschreibung

Die vorliegende Erfindung betrifft ein endoskopisches Instrument nach dem Oberbegriff von Anspruch 1 sowie ein endoskopisches Instrumentensystem mit einem derartigen endoskopischen Instrument.

Endoskopische Operationstechniken haben sich für eine Vielzahl chirurgischer oder diagnostischer Eingriffe durchgesetzt. Dabei wird durch eine natürliche oder eine mit Hilfe einer Inzision geschaffene künstliche Körperöffnung ein endoskopisches Instrumentensystem, das insbesondere eine Endoskopoptik und ein endoskopisches Instrument umfasst, zu einem im Körperinneren, insbesondere in einem körperinneren Hohlraum, gelegenen Zielgebiet geführt, in dem der chirurgische oder diagnostische Eingriff erfolgen soll. Bei vielen Anwendungen hat es sich als vorteilhaft erwiesen, einen starr ausgebildeten Außenschaft durch die Körperöffnung bis in den Hohlraum zu führen, wobei innerhalb des Außenschafts eine starre Endoskopoptik und ein endoskopisches Instrument aufgenommen sind sowie ggf. weitere Kanäle, die etwa zum Einführen weiterer Instrumente oder als Spülkanäle dienen können.

Eine Endoskopoptik, die oft auch als "Endoskop" bezeichnet wird, weist im Fall einer starren Endoskopoptik typischerweise einen starren, lang erstreckten zylindrischen Schaft auf, in dessen distalem, d.h. benutzerfernen, Endbereich ein Endoskopobjektiv zur Erzeugung eines endoskopischen Bildes des Zielgebiets angeordnet ist. Das aufgenommene endoskopische Bild wird typischerweise über einen innerhalb des Schafts angeordneten Bildweiterleiter zum proximalen, d.h. benutzernahen, Ende der Endoskopoptik weitergeleitet. Da in der Regel in dem Hohlraum nicht ausreichend Licht vorhanden ist, ist ferner üblicherweise innerhalb des Schafts der Endoskopoptik ein aus Lichtleitfasern bestehendes Lichtleitsystem angeordnet, um ausreichend Licht an das distale Ende der Endoskopoptik zu transportieren, wo es zur Beleuchtung des Zielgebiets genutzt wird.

Ein endoskopisches Instrument, das zur Benutzung in einem endoskopischen Instrumentensystem der genannten Art ausgebildet ist, weist gemäß einer verbreiteten Bauart ein langerstrecktes Schaftrohr auf, das zur Aufnahme des Schafts der Endoskopoptik ausgebildet ist. Am distalen Ende des Schaftrohrs ist ein Werkzeug zur Durchführung endoskopischer Manipulationen angeordnet, das über ein langerstrecktes Übertragungselement vom proximalen Ende des Schaftrohrs her angesteuert, beispielsweise mittels eines am proximalen Ende angeordneten Handgriffs betätigt werden kann. Der Handgriff und das proximale Ende der Endoskopoptik verbleiben bei dem endoskopischen Eingriff außerhalb der Körperöffnung, während der Außenschaft, das Schaftrohr mit dem Werkzeug und der Schaft der Endoskopoptik zum Zielgebiet geführt werden. Die notwendige Nutzlänge des Außenschafts sowie der Endoskopoptik und des Schaftrohrs mit dem Werkzeug hängt insbesondere von der Art des endoskopischen Eingriffs und von dem genutzten Zugangsweg ab.

Aus US 5,392,765 und US 5,549,541 ist ein Zystoskop bekannt, das einen Außenschaft und ein inneres Element umfasst, wobei das innere Element in einem zentralen Hohlraum des Außenschafts positioniert ist und einen inneren Kanal aufweist, der einen ersten Durchgang umfasst, der zum Aufnehmen einer Endoskopoptik angepasst ist, und einen zweiten Durchgang, der zum Durchführen einer Laserfaser genutzt wird. Weitere Bereiche des inneren Kanals dienen zur Durchleitung von Spülflüssigkeit. Die Führung der Laserfaser ist dabei nicht immer befriedigend.

In EP 0 455 188 A2 ist ein Koagulationsinstrument offenbart, das aus einem Schaft besteht mit einem Kanal zur Durchführung einer Endoskopoptik und einem Kanal zur Durchführung eines flexiblen Koagulations-Spül- und Saugkatheters, welcher distal durch eine proximale Handhabe ablenkbar ist. Dabei sind der obere und untere Schaftbereich durch seitliche Umfangseinschnürungen in zwei auf der Länge ineinander übergehende Kanäle aufgeteilt. Im oberen Kanal kleineren Durchmessers kann eine Optik geführt sein, während durch den unteren Kanal der Koagulations-Spül- und Saugkatheter hindurchgeführt werden kann.

Aus US 2012/0035416 A1 sind endoskopische Instrumente bekannt, wobei ein Überrohr einen offenen peripheren Kanal in einer Wand des Überrohrs aufweist und wobei ein gelenkiger Arm mit mehreren Freiheitsgraden den offenen peripheren Kanal belegt. Das Überrohr weist ein Haupt-Lumen zur Aufnahme eines Schafts einer Endoskopoptik auf, und der periphere Kanal stellt ein axial durchgehendes, in einer Querrichtung an das Haupt-Lumen anschließendes Neben-Lumen dar, wobei das Neben-Lumen eine gegenüber dem Haupt-Lumen kleinere Querschnittsfläche hat und über einen gegenüber dem Haupt-und dem Neben-Lumen verengten Übergangsbereich mit dem Haupt-Lumen verbunden ist. Am distalen Ende des Schaftrohrs ist ein Werkzeug angeordnet, das über ein langerstrecktes Übertragungselement, welches in dem Neben-Lumen aufgenommen ist, von einem proximalen Ende des Schaftrohrs ansteuerbar ist, wobei das Übertragungselement als Zugstange ausgebildet ist, und das Werkzeug mindestens ein bewegliches Werkzeugelement umfasst, das mittels der Zugstange relativ zum Werkzeug beweglich ist. Gemäß US 2010/0145142 A1 ist bei einer minimal invasiven Neurochirurgie-Anordnung ein Schaft vorgesehen, der flexible Wandabschnitte aufweist, die die Einführung von Werkzeugen und Endoskopen mit unterschiedlichen Durchmessern erlauben.

Ein endoskopisches Instrument zur Benutzung in einem endoskopischen Instrumentensystem der eingangs genannten Art wird von der KARL STORZ GmbH & Co. KG unter der Bezeichnung 10377 HF vertrieben. Dabei ist das Schaftrohr zur Aufnahme des zylindrischen Schafts der Endoskopoptik ebenfalls zylindrisch ausgebildet. Am distalen Ende des Schaftrohrs ist ein als Zange ausgebildetes Werkzeug angeordnet, das mittels eines am proximalen Ende des Schaftrohrs angeordneten Handgriffs über ein als Zugstange ausgebildetes Übertragungselement betätigt werden kann. Die Zange befindet sich im Blickfeld einer durch das Schaftrohr geführten Endoskopoptik und ermöglicht somit die Durchführung endoskopischer Manipulationen mit der Zange unter endoskopischer Sicht; ein derartiges Instrument wird daher auch als "optische Zange" bezeichnet. Die Zugstange ist in einem parallel zum Schaftrohr verlaufenden Führungsrohr geführt, das parallel zum Schaftrohr verläuft und an dessen Außenseite angeschweißt ist. Die Herstellung eines derartigen Schaftrohrs mit angeschweißtem Führungsrohr ist relativ aufwändig und kostenintensiv.

Die Endoskopoptik sowie das Schaftrohr, das Werkzeug und der Außenschaft sind häufig für eine mehrfache Verwendung vorgesehen. Nach einem erfolgten Eingriff ist daher vor einer erneuten Verwendung eine Reinigung und Sterilisation des endoskopischen Instrumentensystems erforderlich. Zur Reinigung des Schaftrohrs ist es bekannt, dieses mit einer Reinigungsflüssigkeit zu durchspülen. Die Reinigung des Führungsrohrs ist dabei mit einem relativ hohen Aufwand verbunden.

Es ist Aufgabe der vorliegenden Erfindung, ein endoskopisches Instrument der genannten Art sowie ein endoskopisches Instrumentensystem mit einem derartigen endoskopischen Instrument anzugeben, wobei die oben genannten Nachteile möglichst vermieden werden, insbesondere das hinsichtlich der Herstellung und der Reinigung verbessert ist.

Diese Aufgabe wird durch ein endoskopisches Instrument gemäß Anspruch 1 sowie durch ein endoskopisches Instrumentensystem gemäß Anspruch 8 gelöst. Vorteilhafte Weiterbildungen der Erfindung ergeben sich aus den Unteransprüchen.

Ein erfindungsgemäßes endoskopisches Instrument umfasst ein langerstrecktes Schaftrohr, das ein in axialer Richtung durchgehendes Lumen aufweist, d.h. einen durchgehenden inneren Hohlraum. Das Lumen umfasst zwei ebenfalls axial durchgehende, miteinander in Verbindung stehende Teilbereiche. Ein erster Teilbereich ist zur Aufnahme eines Schafts einer starren Endoskopoptik ausgebildet. Dieser Teilbereich bzw. dieses Teil-Lumen nimmt vorzugsweise den überwiegenden Teil des durchgehenden Hohlraums des Schaftrohrs ein und wird daher im Folgenden als Haupt-Lumen bezeichnet. Da der Schaft einer starren Endoskopoptik in der Regel zylindrisch, insbesondere kreiszylindrisch, ausgebildet ist, ist das Haupt-Lumen ebenfalls im Wesentlichen zylindrisch, insbesondere kreiszylindrisch geformt. Das Haupt-Lumen reicht von einem proximalen bis zu einem distalen Ende des Schaftrohrs. Eine in das Haupt-Lumen des Schaftrohrs eingesetzte Endoskopoptik, deren Schaft eine etwas größere Länge als das Schaftrohr hat, kann daher derart vorgeschoben werden, dass das distale Ende der Endoskopoptik aus dem distalen Ende des Schaftrohrs heraus ragt und gleichzeitig der proximale Endbereich der Endoskopoptik, der einen Anschluss zum Anschließen eines Lichtleitkabels und einen Kameraanschluss oder ein Okular aufweisen kann, proximalseitig außerhalb des Schaftrohrs verbleibt. Die Länge des Schaftrohrs bzw. die Länge des Schafts der Endoskopoptik richtet sich nach der für einen durchzuführenden endoskopischen Eingriff notwendigen Nutzlänge.

Das erfindungsgemäße endoskopische Instrument umfasst weiterhin ein endoskopisches Werkzeug, das an einem distalen Ende des Schaftrohrs angeordnet und mit einem distalen Endbereich des Schaftrohrs verbunden ist. Das Werkzeug kann mit dem Schaftrohr fest oder lösbar verbunden sein. Das Werkzeug ist über ein langerstrecktes Übertragungselement vom proximalen Ende des Schaftrohrs her ansteuerbar. Das Übertragungselement ist als mechanisches Übertragungselement zur Übertragung einer mechanischen Wirkung, nämlich zur Übertragung einer Zugkraft oder - bewegung, eventuell auch einer Schubkraft bzw. -bewegung, ausgebildet. Das Übertragungselement ist ein Bestandteil des endoskopischen Instruments und kann mit diesem fest oder lösbar verbunden sein. Das Übertragungselement ist je nach Art des Werkzeugs mit diesem starr oder gelenkig verbunden bzw. verbindbar.

Erfindungsgemäß weist das Schaftrohr ein in axialer Richtung durchgehendes, in einer ersten Querrichtung zur axialen Richtung an das Haupt-Lumen anschließendes weiteres Teil-Lumen auf, das einen weiteren Teilbereich des durchgehenden Hohlraums des Schaftrohrs bildet. Dieses Teil-Lumen hat eine gegenüber dem Haupt-Lumen kleinere Querschnittsfläche und schließt seitlich an das Haupt-Lumen an und wird daher im Folgenden als Neben-Lumen bezeichnet. Das Neben-Lumen kann im Wesentlichen zylindrisch, insbesondere kreiszylindrisch, geformt sein. Das Neben-Lumen steht über einen gegenüber dem Haupt- und dem Neben-Lumen verengten Übergangsbereich mit dem Haupt-Lumen in Verbindung. Der axial durchgehende Hohlraum kann insbesondere derart ausgestaltet sein, dass das Haupt-Lumen und das Neben-Lumen im Querschnitt jeweils kreisförmig mit unterschiedlichen Durchmessern, jedoch jeweils unter Ausschluss eines Kreisabschnitts, ausgebildet sind, und der Übergangsbereich im Querschnitt durch die beiden ausgeschlossenen, einander zugewandten Kreisabschnitte sowie einen diese verbindenden Bereich gebildet wird. Der Übergangsbereich ist insbesondere in einer zur axialen und zur ersten Querrichtung senkrecht stehenden zweiten Querrichtung verengt, d.h. eine in der zweiten Querrichtung gemessene minimale lichte Weite des Übergangsbereichs ist kleiner als die in derselben Richtung gemessene maximale lichte Weite des Neben-Lumens und des Haupt-Lumens. Das Neben-Lumen nimmt das langerstreckte Übertragungselement auf und ist somit vorzugsweise ebenfalls vom proximalen zum distalen Ende des Schaftrohrs durchgehend ausgebildet.

Das endoskopische Instrument ist insbesondere zur Einführung in einen Außenschaft geeignet, der zum Einführen durch eine natürliche oder künstliche Körperöffnung in einen körperinneren Hohlraum ausgebildet ist. Am proximalen Ende des Schaftrohrs kann beispielsweise ein Handgriff angeordnet bzw. anbringbar sein, der ein Betätigungselement zur Betätigung des Werkzeugs aufweisen kann, das hierfür mit dem Übertragungselement verbunden bzw. verbindbar ist und die Betätigung des mit einem beweglichen Werkzeugelement versehenen Werkzeugs ermöglicht. Ferner können im proximalen Endbereich des Schaftrohrs ein Mechanismus zum Fixieren bzw. Verriegeln einer in das Schaftrohr eingeführten Endoskopoptik und/oder ein Mechanismus zum Fixieren bzw. Verriegeln in einem Außenschaft, in den das Schaftrohr eingeführt worden ist, angeordnet sein. Das endoskopische Instrument kann beispielsweise zur Verwendung mit einem Bronchoskop, einem Ösophagoskop, einem Zystoskop oder einem Uretero-Renoskop ausgebildet sein.

Dadurch, dass das Schaftrohr ein axial durchgehendes, seitlich an das Haupt-Lumen anschließendes und mit diesem über einen Übergangsbereich verbundenes Neben-Lumen zur Aufnahme des Übertragungselements umfasst, wird es ermöglicht, bei einer Spülung des Haupt-Lumens mit einer Reinigungsflüssigkeit zugleich das Neben-Lumen zu spülen und damit auf einfache und sichere Weise auch das Neben-Lumen zu reinigen. Ferner kann dadurch, dass das Lumen des Schaftrohrs ein Haupt-Lumen zur Aufnahme des Schafts der Endoskopoptik und ein Neben-Lumen zur Aufnahme des Übertragungselements zum Ansteuern des Werkzeugs aufweist, die Anbringung eines Führungsrohrs für das Übertragungselement entfallen. Hierdurch können Material und Bearbeitungskosten eingespart werden. Schließlich wird dadurch, dass der Übergangsbereich zwischen dem Neben- und dem Haupt-Lumen verengt ist, eine Führung des Übertragungselements im Neben-Lumen ermöglicht, wodurch die Einführung des Übertragungselements beim Zusammenbau des endoskopischen Instruments erleichtert und die Wirkungsweise des Übertragungselements bei der Benutzung des endoskopischen Instruments verbessert wird.

Vorzugsweise umfasst das endoskopische Instrument nur ein einziges Neben-Lumen und nur einen einzigen Übergangsbereich zwischen dem Neben-Lumen und dem Haupt-Lumen. Hierdurch wird eine besonders einfache und kostengünstig herstellbare sowie sicher zu bedienende und zu reinigende Ausführungsform geschaffen. Es ist aber auch denkbar, dass das endoskopische Instrument mehr als ein Neben-Lumen aufweist, wobei vorzugsweise jedes Neben-Lumen über jeweils einen verengten Übergangsbereich mit dem Haupt-Lumen verbunden ist. Hierdurch wird ein endoskopisches Instrument geschaffen, das eine erweiterte Funktionalität bereitstellt, beispielsweise durch Verwendung mehrerer Werkzeuge, die über jeweils ein Übertragungselement, das in jeweils einem Neben-Lumen aufgenommen ist, vom proximalen Ende her angesteuert werden können.

Das Schaftrohr ist vorzugsweise als Profilrohr aus einem metallischen Werkstoff, etwa Edelstahl, mit einem nicht-kreisförmigen Querschnitt bzw. einem nicht-kreisförmigen Lumen ausgebildet. Ein derartiges Profilrohr ist vorzugsweise nahtlos, beispielsweise durch Strangpressen und/oder Durchziehen, hergestellt. Auf diese Weise wird eine einfache und kostengünstige Herstellung des Schaftrohrs ermöglicht.

Gemäß einer bevorzugten Ausführungsform der Erfindung ist das Schaftrohr mit einer im Wesentlichen einheitlichen Wandstärke ausgebildet. Gemäß dieser Ausführungsform ist somit die Wandstärke des Schaftrohrs, gegebenenfalls von einem proximalen und/oder einem distalen Endabschnitt abgesehen, bei jedem Querschnitt entlang des betreffenden Umfangs jeweils einheitlich. In axialer Richtung gegeneinander versetzte Querschnitte können dabei unterschiedliche Wandstärken aufweisen, vorzugsweise weist das Schaftrohr jedoch in allen Querschnitten die gleiche einheitliche Wandstärke auf, wobei am distalen und/oder am proximalen Ende die Wandstärke abweichen kann, um beispielsweise einen distalen Endanschlag für die Endoskopoptik zu definieren und/oder einen Riegelmechanismus zu befestigen. In besonders vorteilhafter Weise ist das Schaftrohr als Profilrohr aus einem metallischen Werkstoff, etwa Edelstahl, mit einem im Querschnitt nicht-kreisförmigen Lumen und mit einheitlicher Wandstärke ausgebildet. Hierdurch werden eine besonders kostengünstige Herstellung sowie eine günstige Raumausnutzung ermöglicht.

Vorzugsweise ist der verengte Übergangsbereich zumindest bis in einen proximalen Endbereich des Schaftrohrs axial durchgehend ausgebildet. Das Neben-Lumen ist somit im Wesentlichen über die gesamte Länge des Schaftrohrs mit dem Haupt-Lumen verbunden. Dadurch, dass das Neben-Lumen axial durchgehend mit dem Haupt-Lumen in Verbindung steht, wird die Spülung des Neben-Lumens mit Reinigungsflüssigkeit bei einer Spülung des Haupt-Lumens weiter verbessert.

Erfindungsgemäß ist die Verengung des Übergangsbereichs enger als die entsprechende Querdimension des Übertragungselements, d.h. eine minimale lichte Weite des Übergangsbereichs ist geringer als die in einer entsprechenden Richtung, die die zweite, auf der ersten Querrichtung und der axialen Richtung senkrecht stehende Querrichtung sein kann, gemessene maximale Breite des Übertragungselements ist. In dem Fall, dass das Übertragungselement zylindrisch ausgebildet ist, ist die Verengung des Übergangsbereichs somit enger, als es dem Durchmesser des Übertragungselements entspricht. Hierdurch kann sicher verhindert werden, dass das Übertragungselement aus dem Neben-Lumen in das Haupt-Lumen gelangt. Dadurch können die Montage des Instruments und die Führung des Übertragungselements weiter verbessert werden.

Gemäß einer bevorzugten Ausführungsform der Erfindung ist das Übertragungselement im Neben-Lumen in einem Abstand zum Haupt-Lumen geführt, so dass das Übertragungselement dann, wenn ein Schaft einer Endoskopoptik in das Haupt-Lumen eingesetzt ist, in einem Abstand zum Schaft der Endoskopoptik gehalten ist. Insbesondere ist der Übergangsbereich derart bemessen, dass das Übertragungselement von einem zylindrischen Schaft einer Endoskopoptik, der in das Haupt-Lumen eingesetzt ist und der dieses im Querschnitt praktisch ausfüllt, in der ersten Querrichtung einen Abstand aufweist. Hierdurch können eine Berührung und die dadurch verursachte Reibung zwischen dem Schaft der Endoskopoptik und dem Übertragungselement sicher vermieden und somit die Ansteuerung des Werkzeugs über das Übertragungselement weiter verbessert werden. Dies ist besonders vorteilhaft, da das Übertragungselement zur Ansteuerung des Werkzeugs mit mindestens einem beweglichen Werkzeugelement im Neben-Lumen beweglich, nämlich längsverschiebbar, geführt ist.

In besonders bevorzugter Weise ist das Übertragungselement im Neben-Lumen mit einem derartigen Spiel geführt, dass ein für eine Spülung des Neben-Lumens mit Reinigungsflüssigkeit ausreichender Abstand zur Innenwand des Schaftrohrs im Bereich des Neben-Lumens verbleibt. Hierfür kann ein Abstand zwischen dem Übertragungselement und der Innenwand des Schaftrohrs von etwa einem oder wenigen Zehntel Millimetern ausreichend sein. Hierdurch wird die Reinigung des Neben-Lumens, in dem das Übertragungselement verläuft, sowie des Übertragungselements selbst weiter erleichtert.

Das Werkzeug, das am distalen Ende des Schaftrohrs angeordnet ist, kann beispielsweise als Zange, Schere, Stanze, als spreizbarer und zusammenziehbarer Fangkorb oder in ähnlicher Weise ausgebildet sein. Erfindungsgemäß umfasst das Werkzeug mindestens ein bewegliches Werkzeugelement, das über das Übertragungselement betätigt werden kann, wofür das Übertragungselement als Zugstange ausgebildet ist. Die Zugstange kann in vorteilhafter Weise nicht nur zur Ausübung einer Zugkraft, sondern zumindest in begrenztem Maße auch zur Ausübung einer Druck- bzw. Schubkraft geeignet sein. Ein derartiges Zug-/Schubelement wird im Folgenden der Einfachheit halber ebenfalls als "Zugstange" bezeichnet. Dadurch, dass das Werkzeug mindestens ein bewegliches Werkzeugelement umfasst, das mittels des als Zugstange ausgebildeten Übertragungselements betätigbar ist, wird eine Verwendung des endoskopischen Instruments für eine Vielzahl endoskopischer Manipulationen ermöglicht. In besonders vorteilhafter Weise kann hierbei genutzt werden, dass die Zugstange gemäß der oben erwähnten Ausgestaltung des Schaftrohrs in einem Abstand zu einer eingesetzten Endoskopoptik geführt werden kann und somit eine zuverlässige Betätigung des beweglichen Werkzeugelements und die Ausübung hoher Zug- sowie ggf. Schubkräfte auf dieses ermöglicht wird.

Gemäß einer besonders bevorzugten Ausführungsform der Erfindung ist das Werkzeug als Zange oder Schere ausgebildet und umfasst mindestens ein schwenkbares Werkzeugelement. Dieses ist ein Zangenmaulteil bzw. Scherenblatt und um eine im Wesentlichen quer zur Längsachse des Schaftrohrs stehende Querachse schwenkbar gelagert. Hierfür kann die Querachse mit einem distalen Endbereich des Schaftrohrs über einen Halter verbunden sein, wobei die Zugstange gelenkig mit dem mindestens einen schwenkbaren Zangenmaulteil bzw. Scherenblatt verbunden oder verbindbar ist. Durch Betätigung eines am proximalen Ende des endoskopischen Instruments angeordneten Betätigungselements eines Handgriffs kann die Zugstange relativ zum Schaftrohr in axialer Richtung verschoben und dadurch das Werkzeugelement geschwenkt bewegt werden. In besonders vorteilhafter Weise umfasst das Werkzeug zwei gegeneinander wirkende schwenkbare Maulteile bzw. Scherenblätter, die jeweils gelenkig, beispielsweise über jeweils einen Hebel, mit dem distalen Ende der Zugstange verbunden sind. Hierdurch wird ein einfach und sicher zu betätigendes, vielseitig anwendbares endoskopisches Instrument geschaffen, das die Durchführung einer Vielzahl von Gewebemanipulationen in einem körperinneren Hohlraum oder auch die Probenexcision oder die Entfernung von Fremdkörpern unter endoskopischer Sicht ermöglicht.

Weiterhin ist es bevorzugt, dass ein proximaler Endbereich des Schaftrohrs einen Anschluss zur Einleitung von Reinigungsflüssigkeit aufweist. Dieser Anschluss kann beispielsweise am proximalen Ende des Haupt-Lumens angeordnet und mit einem Kupplungsmechanismus zum Anschließen an einen entsprechenden Anschluss einer medizinischen Spülmaschine versehen sein. In besonders bevorzugter Weise ist der Anschluss derart ausgebildet, dass Reinigungsflüssigkeit durch die proximale Öffnung des Haupt-Lumens und somit über einen großen Querschnitt in das Schaftrohr eingeleitet werden kann. Der Kupplungsmechanismus kann mit einem Riegelmechanismus zum Fixieren einer in das Schaftrohr eingesetzten Endoskopoptik kombiniert sein bzw. der Kupplungsmechanismus kann zugleich die Funktion des Riegelmechanismus haben. Die Reinigungsflüssigkeit tritt am proximalen Ende in das Schaftrohr ein und tritt vorzugsweise am distalen Ende wieder aus, wobei das Haupt- und das Neben-Lumen, ggf. gemeinsam mit dem im Neben-Lumen aufgenommenen Übertragungselement, gereinigt werden. Hierdurch wird die Reinigung des endoskopischen Instruments weiter vereinfacht. Die eingeleitete Flüssigkeit kann auch zur Sterilisation des endoskopischen Instruments dienen.

Ein erfindungsgemäßes endoskopisches Instrumentensystem umfasst ein endoskopisches Instrument, das wie zuvor beschrieben ausgebildet ist. Weiter umfasst das endoskopische Instrumentensystem eine starre Endoskopoptik, die in das Haupt-Lumen des Schaftrohrs einsetzbar ist. Das Haupt-Lumen und der Schaft der Endoskopoptik sind vorzugsweise derart aneinander angepasst, dass die Endoskopoptik mit geringem Spiel im Haupt-Lumen geführt ist, wobei der Schaft der Endoskopoptik eine etwas größere Länge als das Schaftrohr hat. Die Endoskopoptik kann daher derart in das Schaftrohr eingeführt werden, dass das distale Ende der Endoskopoptik im Bereich des distalen Endes des Schaftrohrs zu liegen kommt oder um eine kurze Strecke, beispielsweise um etwa einen oder wenige Zentimeter, darüber hinaus ragt, um eine möglichst ungehinderte endoskopische Sicht auf das Werkzeug zu ermöglichen. Der Schaft der Endoskopoptik ist vorzugsweise kreiszylindrisch geformt.

Weiter kann das endoskopische Instrumentensystem einen Außenschaft umfassen, in den das endoskopische Instrument eingesetzt werden kann und der hierfür einen entsprechenden durchgehenden Hohlraum aufweist. Dabei ist die Länge des Außenschafts derart gewählt, dass das Werkzeug des endoskopischen Instruments aus einer distalen Öffnung des Außenschafts herausragt und mit einem Betätigungselement bzw. Versorgungselement, das proximalseitig des Außenschafts angeordnet ist, angesteuert werden kann. Der Außenschaft ist insbesondere atraumatisch ausgebildet und kann beispielsweise eine einseitig über die distale Öffnung vorstehende Lippe zur Schaffung eines Sicht- und Arbeitsraums für die Endoskopoptik und das Werkzeug aufweisen. Der Außenschaft kann beispielsweise einen kreisförmigen, einen ovalen oder auch einen aus zwei Halbkreisen mit dazwischenliegenden Geradenstücken gebildeten Querschnitt haben, wobei ein verbleibender Zwischenraum zwischen dem in den Außenschaft eingesetzten Schaftrohr des endoskopischen Instruments zur Spülung eines Operationsgebiets, in dem mit dem Werkzeug des endoskopischen Instruments endoskopische Manipulationen durchgeführt werden, oder auch zur Aufnahme weiterer endoskopischer Werkzeuge dienen.

Das endoskopische Instrumentensystem kann weitere Komponenten umfassen, beispielsweise weitere Werkzeuge, Spülanschlüsse, Versorgungseinrichtungen zur Bereitstellung von Beleuchtungslicht, Spülflüssigkeit oder elektrischer oder Lichtenergie für das endoskopische Werkzeug, sowie Anzeige- und Auswerteeinrichtungen für ein aufgenommenes endoskopisches Bild.

Bei der Durchführung eines endoskopischen Eingriffs mit dem erfindungsgemäßen endoskopischen Instrumentensystem wird eine Endoskopoptik in das endoskopische Instrument, das das Werkzeug umfasst, eingeführt und darin in einer Position fixiert, so dass das Werkzeug mit der Endoskopoptik beobachtet werden kann. Zuvor kann ein Handgriff, der zur Betätigung des Werkzeugs mit dem im Schaftrohr aufgenommenen Übertragungselement zusammenwirkt, am proximalen Endbereich des Schaftrohrs befestigt worden sein. Das endoskopische Instrument mit der Endoskopoptik wird in einen Außenschaft eingeführt und in einer geeigneten Position, in der beispielsweise eine atraumatische Lippe ausreichend weit über das distale Ende der Endoskopoptik hinausragt, um einen Sicht- und Arbeitsraum zu schaffen, fixiert. Der Außenschaft wird sodann mit dem endoskopischen Instrument und der Endoskopoptik durch eine Körperöffnung zum Zielgebiet, wo der endoskopische Eingriff durchgeführt werden soll, eingeführt. Die genannten Schritte können auch in anderer Reihenfolge oder ggf. nach Einführung des Außenschafts durch die Körperöffnung erfolgen. Nach Beendigung des Eingriffs wird der Außenschaft aus der Körperöffnung herausgezogen und das endoskopische Instrument und die Endoskopoptik jeweils gelöst und aus dem Außenschaft bzw. dem Schaftrohr entfernt; auch diese Schritte können ggf. in anderer Reihenfolge durchgeführt werden. Zur Aufbereitung wird das Haupt-Lumen des endoskopischen Instruments mit Reinigungsflüssigkeit beaufschlagt, die in das Neben-Lumen eindringt und dieses ebenfalls durchspült. Vorzugsweise wird hierfür ein Spülanschluss des endoskopischen Instruments mit einem entsprechenden Anschluss einer medizinischen Spülmaschine verbunden und das endoskopische Instrument in dieser gereinigt. Der Außenschaft und die Endoskopoptik sowie ggf. der Handgriff werden in üblicher Weise gereinigt. Weiter erfolgt eine Sterilisation, beispielsweise durch Autoklavieren, in bekannter Weise.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind. Weitere Aspekte der Erfindung ergeben sich aus der nachfolgenden Beschreibung eines bevorzugten Ausführungsbeispiels und der beigefügten Zeichnung. Es zeigen:
Fig. 1 den distalen Endbereich eines endoskopischen Instruments gemäß einem ersten Ausführungsbeispiel der Erfindung in einer perspektivischen Ansicht;
Fig. 2 das endoskopische Instrument gemäß Fig. 1, jedoch ohne den Halter des Werkzeugs;
Fig. 3 das endoskopische Instrument gemäß Fig. 2 in einer Gesamtansicht mit angesetztem Handgriff;
Fig. 4 den distalen Endbereich des endoskopischen Instruments wie in Fig. 2, jedoch mit einer in das Schaftrohr eingeführten Endoskopoptik;
Fig. 5 den distalen Endbereich des endoskopischen Instruments wie in Fig. 1, jedoch in ein Außenrohr eingesetzt;
Fig. 6 ein endoskopisches Instrument gemäß einem zweiten Ausführungsbeispiel der Erfindung in einer Fig. 4 entsprechenden Ansicht.

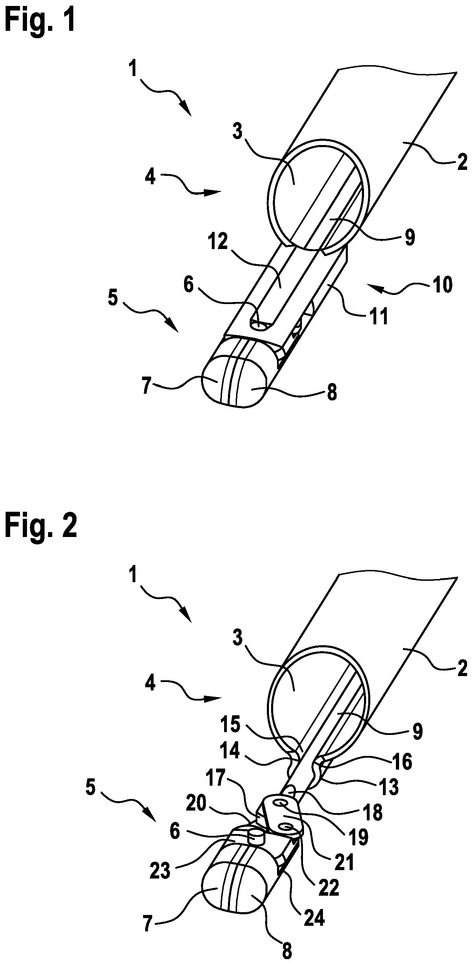

In Fig. 1 ist der distale Endbereich eines gemäß einem ersten Ausführungsbeispiel der Erfindung ausgebildeten endoskopischen Instruments 1 in einer schräg aus distaler Richtung gesehenen Ansicht dargestellt. Das endoskopische Instrument 1 weist ein Schaftrohr 2 auf, das einen durchgehenden, im Wesentlichen zylindrischen Hohlraum umschließt, der hier als Haupt-Lumen 3 bezeichnet wird. Das Haupt-Lumen 3 mündet distalseitig in einer Öffnung, die zu einer Längsachse des Schaftrohrs 2 schräg gestellt ist. Am distalen Ende 4 des Schaftrohrs 2 ist das als Zange 5, beispielsweise als Fass- oder Biopsiezange, ausgebildete Werkzeug angeordnet. Die Zange 5 umfasst zwei um eine Querachse 6 schwenkbare, gegeneinander wirkende Maulteile 7, 8, die jeweils gelenkig an einer unterhalb des Haupt-Lumens 3 verlaufenden Zugstange 9 angelenkt sind. Die Querachse 6 wird von einem als Gabel 10 ausgebildeten Halter am distalen Endbereich des Schaftrohrs 2 gehalten. Die Gabel 10 besteht aus einem Hauptteil 11, das den in Fig. 1 nicht im Einzelnen dargestellten Gelenkmechanismus, über den die Maulteile 7, 8 mit der Zugstange 9 verbunden sind, umschließt und in das die Querachse 6 eingesetzt ist. In proximaler Richtung wird die Querachse 6 durch den Einsatz 12 abgestützt. Das Hauptteil 11 der Gabel 10 kann ein- oder mehrteilig ausgebildet sein. Die Gabel 10, zumindest das Hauptteil 11, ist durch Schweißen am distalen Endbereich des Schaftrohrs 2 befestigt.

Wie in Fig. 2 in einer der Fig. 1 entsprechenden Ansicht, bei der jedoch die Gabel 10 weggelassen ist, dargestellt ist, ist das Schaftrohr 2 als Profilrohr ausgebildet, das einen nicht-kreisförmigen Querschnitt aufweist und unterhalb des durchgehenden, im Wesentlichen zylindrischen Haupt-Lumens 3 ein durch die Ausbauchung 13 gebildetes Neben-Lumen 14 umschließt, das ebenfalls axial durchgehend ausgebildet ist und in dem die Zugstange 9 aufgenommen ist. In einem Übergangsbereich 15 zwischen dem Haupt-Lumen 3 und dem Neben-Lumen 14 bildet die Wand des Schaftrohrs 2 eine Verengung 16, deren lichte Weite geringer ist als der Durchmesser der zylindrischen Zugstange 9, so dass diese innerhalb des Neben-Lumens 14 gehalten und sicher geführt ist. Das Neben-Lumen 14 ist teilweise zylindrisch ausgebildet, wobei der Radius des Zylinders derart gewählt ist, dass zwischen der im Neben-Lumen 14 aufgenommenen Zugstange 9 und der Wand des Schaftrohrs 2 im Bereich der Ausbauchung 13 ein Zwischenraum verbleibt, der nicht nur eine reibungsarme Längsverschiebbarkeit der Zugstange 9, sondern auch den Durchtritt von Reinigungsflüssigkeit zwischen der Zugstange 9 und der Wand des Schaftrohrs 2 ermöglicht. Die distale Öffnung des Schaftrohrs 2 steht im Bereich der Ausbauchung 13 im Wesentlichen senkrecht zur Längsachse des Schaftrohrs 2.

Das distale Ende der Zugstange 9 wird durch eine Platte 17 gebildet, wobei der Übergang vom zylindrischen Querschnitt der Zugstange 9 zur Platte 17 durch eine Abschrägung 18 vermittelt wird. An der Platte 17 setzen beidseitig je ein mit einem Achsstift 19 gelenkig gelagerter Hebel 20, 21 an. Die Hebel 20, 21 sind wiederum über jeweils einen Achsstift 22 mit einem jeweiligen plattenförmigen Lagerabschnitt 23, 24 der Maulteile 7, 8 verbunden. Die Maulteile 7, 8 sind mittels ihrer Lagerabschnitte 23, 24 an der Querachse 6 schwenkbar gelagert. Da die Querachse 6 am distalen Ende des Schaftrohrs 2 gehalten ist, können die Maulteile 7, 8 durch eine Längsbewegung der Zugstange 9 gespreizt und wieder geschlossen werden.

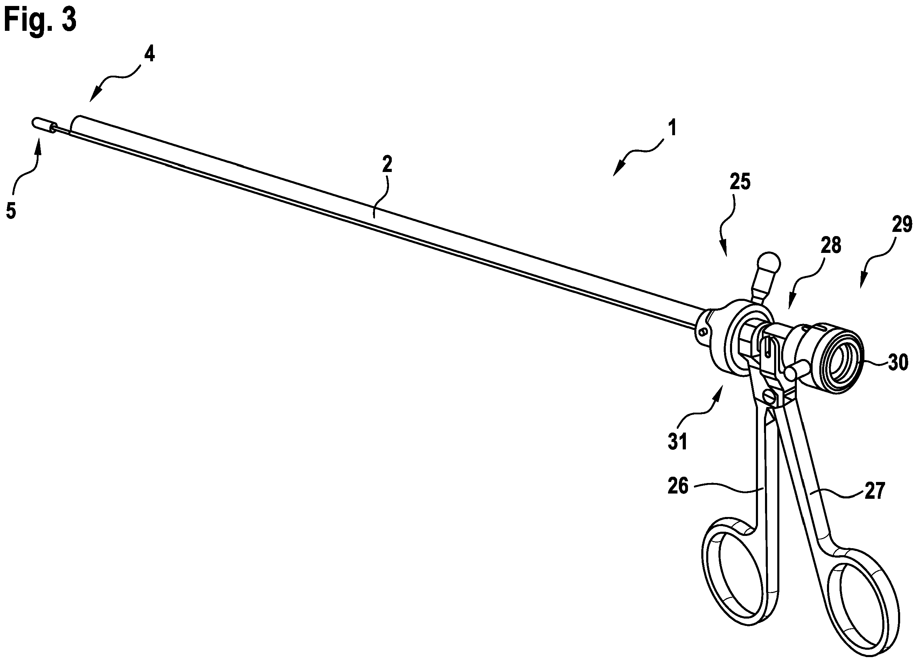

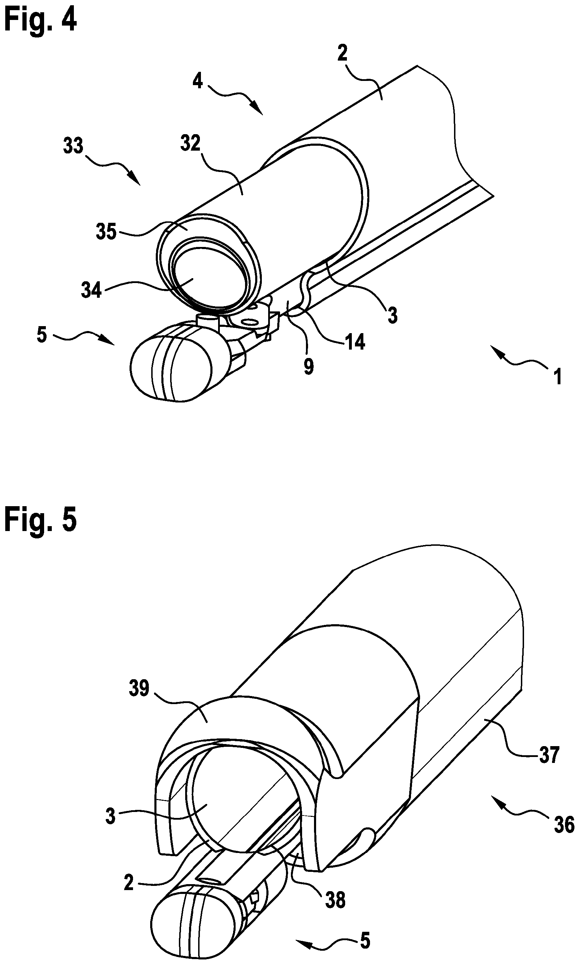

Wie in Fig. 3 in einer schräg aus proximaler Richtung gesehenen Ansicht des endoskopischen Instruments 1 gezeigt ist, ist zum Längsverschieben der Zugstange 9 und damit zum Betätigen der Maulteile 7, 8 der Zange 5 am proximalen Ende 25 des Schaftrohrs 2 des endoskopischen Instruments 1 ein Handgriff anbringbar. Der Handgriff besteht aus einem feststehenden Griffteil 26, das lösbar mit dem Schaftrohr 2 verbunden ist, und einem gegenüber dem feststehenden Griffteil 26 schwenkbar gelagerten Griffteil 27. Das Griffteil 27 ist über einen lösbaren Verbindungsmechanismus 28 mit dem proximalen Endbereich der Zugstange 9 (s. Fig. 2) verbunden. Zur Erleichterung der Handhabung weisen die Griffteile 26, 27 jeweils einen Griffring auf. Weiterhin ist in Fig. 3 zu erkennen, dass im proximalen Endbereich des Schaftrohrs 2 ein erster Riegelmechanismus 29 angeordnet ist, der eine proximalseitige Verlängerung des Haupt-Lumens 3 (s. Fig. 1, 2) umschließt und mit Hilfe dessen eine in diese und in das Haupt-Lumen 3 des Schaftrohrs 2 eingesetzte Endoskopoptik befestigt und in einer geeigneten Position (s. Fig. 4) verriegelt werden kann. Die proximalseitige Öffnung der Verlängerung des Haupt-Lumens 3 dient zugleich als Spülanschluss 30 für die Einleitung von Reinigungsflüssigkeit. Hierfür ist der erste Riegelmechanismus 29 auch als Kupplung zum Anschließen an eine entsprechende Einrichtung einer medizinischen Spülmaschine ausgebildet. Hierdurch kann Reinigungsflüssigkeit in das Haupt-Lumen 3 und über den Übergangsbereich 15 auch in das Neben-Lumen 14 des Schaftrohrs 2 eingeleitet werden, so dass Verunreinigungen in distaler Richtung ausgespült werden. Weiter ist im proximalen Endbereich ein zweiter Riegelmechanismus 31 vorgesehen, mit dessen Hilfe das endoskopische Instrument 1, wenn es in einen Außenschaft (s. Fig. 5) eingesetzt ist, in diesem in einer geeigneten Position fixiert werden kann.

In Fig. 4 ist wie in Fig. 2 das distale Ende des Schaftrohrs 2 des endoskopischen Instruments 1 gezeigt, jedoch mit in das Haupt-Lumen 3 eingesetztem Schaft 32 einer Endoskopoptik 33. Die Gabel 10 (s. Fig. 1) ist nicht gezeigt. Wie in Fig. 4 dargestellt, umfasst die distale Endfläche des Schafts 32 der Endoskopoptik 33 eine Lichteintrittsfläche 34 des optischen Systems der Endoskopoptik 33 und eine Lichtaustrittsfläche 35, in der die Lichtleitfasern des Lichtleitsystems für das Beleuchtungslicht münden. Die Endoskopoptik 33 umfasst ein Okular bzw. einen Kameraanschluss sowie einen Anschluss für Beleuchtungslicht, die am proximalen Ende des Schafts 32 angeordnet sind und die außerhalb des Schaftrohrs 2 verbleiben (nicht dargestellt). In dem in Fig. 4 gezeigten Beispiel ist die Endfläche der Endoskopoptik 33 zur Längsachse des Schafts 32 geneigt, d.h. die Endoskopoptik 33 ist eine Schrägblickoptik. Dies ermöglicht es, dass die Zange 5 günstig im Blickfeld der Endoskopoptik 33 angeordnet ist, so dass mit der Zange 5 vorgenommene Manipulationen mit Hilfe der Endoskopoptik 33 beobachtet werden können. Hierfür ist der Schaft 32 der Endoskopoptik 33 geringfügig über die distale Öffnung des Schaftrohrs 2 hinausgeschoben. In dieser Position kann die Endoskopoptik 33 mittels des ersten Riegelmechanismus 29 im Schaftrohr 2 fixiert werden (s. Fig. 3).

In Fig. 5 ist der distale Endbereich des Schaftrohrs 2 mit der Zange 5 wie in Fig. 1 dargestellt, wobei jedoch das Schaftrohr 2 in einen Außenschaft 36 eingesetzt ist. Der Außenschaft 36 umfasst ein langerstrecktes Außenrohr 37, das einen durchgehendenden Hohlraum zur Aufnahme des Schaftrohrs 2 ausbildet und das einen aus zwei Halbkreisen mit dazwischenliegenden Geradenstücken gebildeten Querschnitt aufweist. Ein verbleibender Zwischenraum 38 zwischen den Außenrohr 37 und dem Schaftrohr 2 kann beispielsweise zur Spülung des Operationsgebiets, in dem mit der Zange 5 die endoskopischen Manipulationen durchgeführt werden, genutzt werden. Am distalen Ende des Außenrohrs 37 ist auf dieses eine atraumatisch abgerundete, hochstehende Lippe 39 aufgesetzt, die zur Schaffung eines Hohlraums zur Durchführung der endoskopischen Manipulationen mit der Zange 5 und zur Ermöglichung einer freien Sicht aus der wie in Fig. 4 eingeführten Endoskopoptik, die in Fig. 5 nicht gezeigt ist, dient. In einer hierfür geeigneten Position kann das endoskopische Instrument 1 mittels des zweiten Riegelmechanismus innerhalb des Außenschafts 36 fixiert werden (s. Fig. 3).

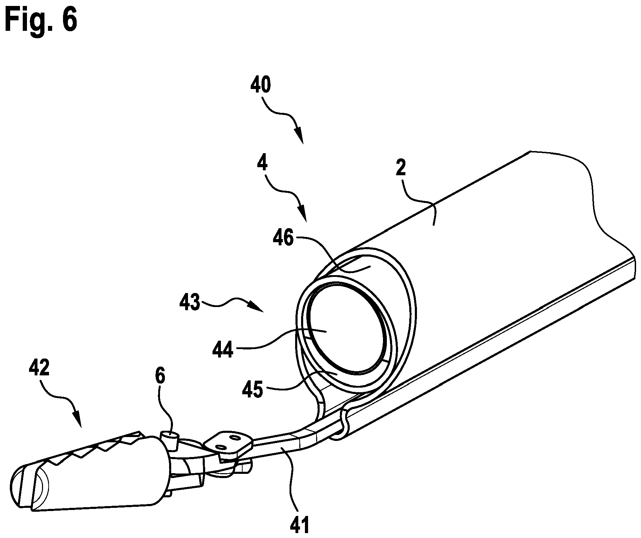

In Fig. 6 ist eine zweite Ausführungsform der Erfindung gezeigt, wobei wie in Fig. 4 eine Endoskopoptik in das Schaftrohr 2 eingeführt ist. Bei dem endoskopischen Instrument 40 gemäß Fig. 6 ist die Zugstange 41 abgeflacht ausgebildet und in ihrem distalen Endbereich gegenüber der Längsachse des Schaftrohrs 2 nach oben, d.h. zur Längsachse hin, abgewinkelt. Dies ermöglicht es, dass das Werkzeug, das gemäß Fig. 6 als Fasszange 42 ausgebildet ist, nahe einer Mittelachse des Schaftrohrs 2 zu liegen kommt, so dass dieses in vorteilhafter Weise mit einer Endoskopoptik 43, deren Lichteintrittsfläche 44 und Lichtaustrittsfläche 45 senkrecht zur Längsachse des Schafts 46 der Endoskopoptik 43 stehen und die somit als Geradeausblickoptik ausgebildet ist, beobachtet werden kann. Im Übrigen ist das in Fig. 6 gezeigte Ausführungsbeispiel wie das zuvor beschriebene und in den Figuren 1 bis 5 dargestellte ausgebildet. Der Halter, mit dem die Querachse 6 am distalen Ende 4 des Schaftrohrs 2 gehalten ist, ist in Fig. 6 nicht gezeigt.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskopisches Instrument
- 2: Schaftrohr
- 3: Haupt-Lumen
- 4: Distales Ende
- 5: Zange
- 6: Querachse
- 7: Maulteil
- 8: Maulteil
- 9: Zugstange
- 10: Gabel
- 11: Hauptteil
- 12: Einsatz
- 13: Ausbauchung
- 14: Neben-Lumen
- 15: Übergangsbereich
- 16: Verengung
- 17: Platte
- 18: Abschrägung
- 19: Achsstift
- 20: Hebel
- 21: Hebel
- 22: Achsstift
- 23: Lagerabschnitt
- 24: Lagerabschnitt
- 25: Proximales Ende
- 26: Griffteil
- 27: Griffteil
- 28: Verbindungsmechanismus
- 29: Riegelmechanismus
- 30: Spülanschluss
- 31: Riegelmechanismus
- 32: Schaft
- 33: Endoskopoptik
- 34: Lichteintrittsfläche
- 35: Lichtaustrittsfläche
- 36: Außenschaft
- 37: Außenrohr
- 38: Zwischenraum
- 39: Lippe
- 40: Endoskopisches Instrument
- 41: Zugstange
- 42: Fasszange
- 43: Endoskopoptik
- 44: Lichteintrittsfläche
- 45: Lichtaustrittsfläche
- 46: Schaft

## Patentansprüche

1. Endoskopisches Instrument mit einem langerstreckten Schaftrohr (2), das ein axial durchgehendes im Wesentlichen zylindrisches Haupt-Lumen (3) zur Aufnahme eines Schafts (32, 46) einer Endoskopoptik (33, 43) aufweist, mit einem Werkzeug, das an einem distalen Ende (4) des Schaftrohrs (2) angeordnet und mit einem distalen Endbereich des Schaftrohrs (2) verbunden ist, und mit einem langerstreckten Übertragungselement, wobei das Werkzeug über das Übertragungselement von einem proximalen Ende (25) des Schaftrohrs (2) ansteuerbar ist, wobei das Übertragungselement als Zugstange (9) ausgebildet ist und das Werkzeug mindestens ein bewegliches Werkzeugelement umfasst, das mittels der Zugstange (9) relativ zum Werkzeug bewegbar ist, und wobei das Schaftrohr (2) ein axial durchgehendes, in einer Querrichtung an das Haupt-Lumen (3) anschließendes Neben-Lumen (14) aufweist, das eine gegenüber dem Haupt-Lumen kleinere Querschnittsfläche hat, das über einen gegenüber dem Haupt- und dem Neben-Lumen verengten Übergangsbereich (15) mit dem Haupt-Lumen (3) verbunden ist und das das Übertragungselement aufnimmt, wobei der verengte Übergangsbereich (15) enger ist, als einer entsprechenden Querdimension des Übertragungselements entspricht.

2. Endoskopisches Instrument nach Anspruch 1, **dadurch gekennzeichnet, dass** das Schaftrohr (2) mit einer einheitlichen Wandstärke ausgebildet ist.

3. Endoskopisches Instrument nach Anspruch 1 oder 2, **dadurch gekennzeichnet, dass** der verengte Übergangsbereich (15) axial durchgehend ausgebildet ist.

4. Endoskopisches Instrument nach einem der vorhergehenden Ansprüche, **dadurch gekennzeichnet, dass** der verengte Übergangsbereich (15) derart bemessen ist, dass das Übertragungselement von einem zylindrischen Schaft (32, 46) einer in das Haupt-Lumen (3) eingesetzten und dieses im Querschnitt praktisch ausfüllenden Endoskopoptik (33, 43) in einer Querrichtung, in der das Neben-Lumen (14) an das Haupt-Lumen (3) anschließt, einen Abstand aufweist.

5. Endoskopisches Instrument nach einem der vorhergehenden Ansprüche, **dadurch gekennzeichnet, dass** das Übertragungselement in dem Neben-Lumen (14) mit einem Abstand zwischen dem Übertragungselement und der Innenwand des Schaftrohrs (2) von etwa einem oder wenigen Zehntel Millimetern geführt ist.

6. Endoskopisches Instrument nach einem der vorhergehenden Ansprüche, **dadurch gekennzeichnet, dass** das Werkzeug als Zange (5, 42) oder Schere ausgebildet ist und mindestens ein um eine Querachse (6) schwenkbares Zangen- bzw. Scherenteil umfasst, das gelenkig mit einem distalen Ende der Zugstange (9) verbunden ist.

7. Endoskopisches Instrument nach einem der vorhergehenden Ansprüche, **dadurch gekennzeichnet, dass** ein proximaler Endbereich des Schaftrohrs (2) einen Anschluss zur Einleitung einer Reinigungsflüssigkeit aufweist.

8. Endoskopisches Instrumentensystem mit einem endoskopischen Instrument (1, 40) nach einem der vorhergehenden Ansprüche und mit einer starren Endoskopoptik (33, 43), die in das Haupt-Lumen (3) des Schaftrohrs (2) des endoskopischen Instruments (1, 40) einsetzbar ist.

## Claims

1. Endoscopic instrument with an elongate shaft tube (2) which has an axially continuous and substantially cylindrical main lumen (3) for receiving a shaft (32, 46) of an endoscope lens system (33, 43), with a tool which is arranged at a distal end (4) of the shaft tube (2) and is connected to a distal end region of the shaft tube (2), and with an elongate transmission element, wherein the tool can be controlled via the transmission element from a proximal end (25) of the shaft tube (2), wherein the transmission element is designed as a pull rod (9) and the tool comprises at least one movable tool element that is movable relative to the tool by means of the pull rod (9), and wherein the shaft tube (2) has an axially continuous secondary lumen (14), which adjoins the main lumen (3) in a transverse direction, has a smaller cross-sectional area than the main lumen, is connected to the main lumen (3) via a transition region (15), narrowed in relation to the main lumen and the secondary lumen, and receives the transmission element, wherein the narrowed transition region (15) is narrower than a corresponding transverse dimension of the transmission element.

2. Endoscopic instrument according to Claim 1, **characterized in that** the shaft tube (2) is configured with a uniform wall thickness.

3. Endoscopic instrument according to Claim 1 or 2, **characterized in that** the narrowed transition region (15) is axially continuous.

4. Endoscopic instrument according to one of the preceding claims, **characterized in that** the narrowed transition region (15) is dimensioned in such a way that the transmission element is at a distance from a cylindrical shaft (32, 46) of an endoscope lens system (33, 43) inserted into the main lumen (3) and practically filling the latter in cross section, in a transverse dimension in which the secondary lumen (14) adjoins the main lumen (3).

5. Endoscopic instrument according to one of the preceding claims, **characterized in that** the transmission element is guided in the secondary lumen (14) with a distance of about one millimetre or several tenths of a millimetre between the transmission element and the inner wall of the shaft tube (2).

6. Endoscopic instrument according to one of the preceding claims, **characterized in that** the tool is configured as forceps (5, 42) or scissors and comprises at least one forceps or scissor part which is pivotable about a transverse stub (6) and which is connected in an articulated manner to a distal end of the pull rod (9).

7. Endoscopic instrument according to one of the preceding claims, **characterized in that** a proximal end region of the shaft tube (2) has an attachment piece for the introduction of a cleaning liquid.

8. Endoscopic instrument system with an endoscopic instrument (1, 40) according to one of the preceding claims and with a rigid endoscope lens system (33, 43) which can be inserted into the main lumen (3) of the shaft tube (2) of the endoscopic instrument (1, 40).

## Revendications

1. Instrument endoscopique comprenant une tige tubulaire allongée (2) qui présente une lumière principale (3) essentiellement cylindrique s'étendant axialement pour recevoir une tige (32, 46) d'une optique d'endoscope (33, 43), un outil qui est disposé à une extrémité distale (4) de la tige tubulaire (2) et qui est connecté à une région d'extrémité distale de la tige tubulaire (2), et un élément de transfert allongé, l'outil pouvant être commandé par le biais de l'élément de transfert depuis une extrémité proximale (25) de la tige tubulaire (2), l'élément de transfert étant réalisé sous forme de tige de traction (9) et l'outil comprenant au moins un élément d'outil mobile qui peut être déplacé par rapport à l'outil au moyen de la tige de traction (9) et la tige tubulaire (2) présentant une lumière secondaire (14) s'étendant axialement, se raccordant à la lumière principale (3) dans une direction transversale, laquelle présente une surface en section transversale inférieure à celle de la lumière principale, est connectée à la lumière principale (3) par le biais d'une région de transition (15) rétrécie par rapport à la lumière principale et à la lumière secondaire, et reçoit l'élément de transfert, la région de transition (15) rétrécie étant plus étroite qu'une dimension transversale correspondante de l'élément de transfert.

2. Instrument endoscopique selon la revendication 1, **caractérisé en ce que** la tige tubulaire (2) est réalisée avec une épaisseur de paroi uniforme.

3. Instrument endoscopique selon la revendication 1 ou 2, **caractérisé en ce que** la région de transition rétrécie (15) est réalisée de manière à s'étendre axialement.

4. Instrument endoscopique selon l'une quelconque des revendications précédentes, **caractérisé en ce que** la région de transition rétrécie (15) est dimensionnée de telle sorte que l'élément de transfert est espacé d'une tige cylindrique (32, 46) d'une optique d'endoscope (33, 43) insérée dans la lumière principale (3) et remplissant pratiquement celle-ci en section transversale dans une direction transversale, dans laquelle la lumière secondaire (14) se raccorde à la lumière principale (3).

5. Instrument endoscopique selon l'une quelconque des revendications précédentes, **caractérisé en ce que** l'élément de transfert est guidé dans la lumière secondaire (14) à une distance entre l'élément de transfert et la paroi interne de la tige tubulaire (2) d'environ un dixième de millimètre ou de quelques dixièmes de millimètres.

6. Instrument endoscopique selon l'une quelconque des revendications précédentes, **caractérisé en ce que** l'outil est réalisé sous forme de pince (5, 42) ou de ciseaux et comprend au moins une partie de pince ou de ciseau pouvant pivoter autour d'un axe transversal (6), laquelle est connectée de manière articulée à une extrémité distale de la tige de traction (9).

7. Instrument endoscopique selon l'une quelconque des revendications précédentes, **caractérisé en ce qu'**une région d'extrémité proximale de la tige tubulaire (2) présente un raccord pour l'introduction d'un liquide de nettoyage.

8. Système d'instrument endoscopique comprenant un instrument endoscopique (1, 40) selon l'une quelconque des revendications précédentes, et comprenant une optique d'endoscope rigide (33, 43) qui peut être insérée dans la lumière principale (3) de la tige tubulaire (2) de l'instrument endoscopique (1, 40).
